(19) Europäisches Patentamt
European Patent Office
Office européen des brevets

(11) Veröffentlichungsnummer: **0 012 180**
**B1**

(12) # EUROPÄISCHE PATENTSCHRIFT

(45) Veröffentlichungstag der Patentschrift: **23.06.82**

(51) Int. Cl.³: **C 07 D 211/90**, C 07 D 401/04, A 61 K 31/435, A 61 K 31/44 // (C07D401/04, 211/90, 213/04)

(21) Anmeldenummer: **79104045.4**

(22) Anmeldetag: **19.10.79**

(54) **Neue 1,4-Dihydropyridinverbindungen, Verfahren zu ihrer Herstellung, die genannten Verbindungen enthaltende Arzneimittel und Verfahren zur Herstellung dieser Arzneimittel.**

(30) Priorität: **31.10.78 DE 2847236**

(43) Veröffentlichungstag der Anmeldung: **25.06.80 Patentblatt 80/13**

(45) Bekanntmachung des Hinweises auf die Patenterteilung: **23.06.82 Patentblatt 82/25**

(84) Benannte Vertragsstaaten: **AT BE CH DE FR GB IT NL SE**

(56) Entgegenhaltungen:
**DE-A1-2 508 181**
**US-A-3 966 946**
**US-A-3 974 275**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Wehinger, Egbert, Dr., Donnenbergerstrasse 90, D-5620 Velbert 15 (DE)**
Erfinder: **Bossert, Friedrich, Dr., Claudiusweg 7, D-5600 Wuppertal 1 (DE)**
Erfinder: **Vater, Wulf, Dr., Menchendahlerstrasse 23, D-5090 Leverkusen 3 (DE)**
Erfinder: **Stoepel, Kurt, Dr., In den Birken 69, D-5600 Wuppertal 1 (DE)**

**EP 0 012 180 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Neue 1,4-Dihydropyridinverbindungen, Verfahren zu ihrer Herstellung, die genannten Verbindungen enthaltende Arzneimittel und Verfahren zur Herstellung dieser Arzneimittel

Die vorliegende Erfindung betrifft neue 1,4-Dihydropyridine mit funktionalisierten Estergruppen, mehrere Verfahren zu ihrer Herstellung sowie diese Verbindungen enthaltende Arzneimittel, insbesondere kreislaufbeeinflussende Mittel.

Es ist bereits bekanntgeworden, daß man 1,4-Dihydro-2,6-dimethyl-4-phenyl-pyridin-3,5-dicarbonsäurediäthylester erhält, wenn man Benzylidenacetessigsäureäthylester mit $\beta$-Amino-crotonsäureäthylester oder Acetessigsäureäthylester und Ammoniak umsetzt (Knoevenagel, Ber. dtsch. chem. Ges., 31, 743 [1898]).

Weiterhin ist bekannt, daß bestimmte 1,4-Dihydropyridine interessante pharmakologische Eigenschaften aufweisen (F. Bossert, W. Vater, Naturwissenschaften, 58, 578 [1971]).

Die vorliegende Erfindung betrifft neue 1,4-Dihydropyridine mit funktionalisierten Estergruppen der allgemeinen Formel I

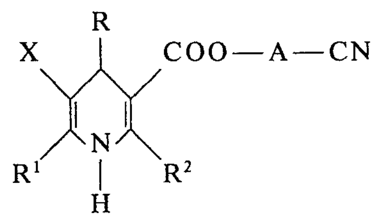

(I)

in welcher

R für einen Phenylrest steht, der durch Trifluormethyl, Nitro, Halogen oder Cyano substituiert ist oder für einen Pyridylrest steht,

$R^1$ und $R^2$ gleich oder verschieden sind und jeweils für Methyl oder Ethyl stehen,

A für eine geradkettige oder verzweigte Alkylengruppe mit bis zu 4 Kohlenstoffatomen steht,

X für die Gruppe $-COOR^7$ steht, wobei $R^7$ einen geradkettigen, verzweigten, cyclischen, gesättigten oder ungesättigten Kohlenwasserstoffrest mit bis zu 6 Kohlenstoffatomen bedeutet, der gegebenenfalls durch 1 Sauerstoff- oder Schwefelatom in der Kette unterbrochen ist und der gegebenenfalls substituiert ist durch Trifluormethyl, Phenyl oder Di($C_1-C_4$)-alkylamino, oder für die Gruppe $-COO-A'-CN$ steht, wobei diese Gruppe mit $-COO-A-CN$ gleich oder verschieden ist und A' für eine geradkettige oder verzweigte Alkylengruppe mit bis zu 4 Kohlenstoffatomen steht.

Es wurde gefunden, daß man die 1,4-Dihydropyridinderivate der allgemeinen Formel I

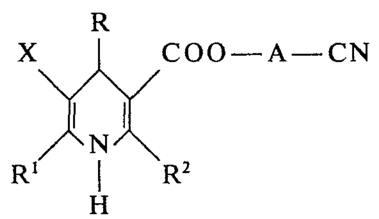

(I)

in welcher

R für einen Phenylrest steht, der durch Trifluormethyl, Nitro, Halogen oder Cyano substituiert ist oder für einen Pyridylrest steht,

$R^1$ und $R^2$ gleich oder verschieden sind und jeweils für Methyl oder Ethyl stehen,

A für eine geradkettige oder verzweigte Alkylengruppe mit bis zu 4 Kohlenstoffatomen steht,

X für die Gruppe $-COOR^7$ steht, wobei $R^7$ einen geradkettigen, verzweigten, cyclischen, gesättigten oder ungesättigten Kohlenwasserstoffrest mit bis zu 6 Kohlenstoffatomen bedeutet, der gegebenenfalls durch 1 Sauerstoff- oder Schwefelatom in der Kette unterbrochen ist und der gegebenenfalls substituiert ist durch Trifluormethyl, Phenyl oder Di-($C_1-C_4$-alkylamino, oder für die Gruppe $-COO-A'-CN$ steht, wobei diese Gruppe mit $-COO-A-CN$ gleich oder verschieden ist und A' für eine geradkettige oder verzweigte Alkylengruppe mit bis zu 4 Kohlenstoffatomen steht,

erhält, wenn man

A) Ylidenverbindungen der Formel II

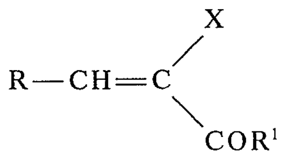

(II)

in welcher R, $R^1$ und X die oben angegebene Bedeutung haben, mit Enaminocarbonsäureestern der Formel III

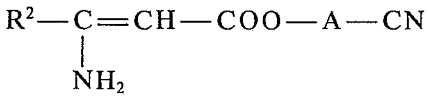

(III)

in welcher $R^2$ und A die oben angegebene Bedeutung haben, gegebenenfalls in Gegenwart inerter organischer Lösungsmittel umsetzt oder

B) Ylidenverbindungen der Formel II

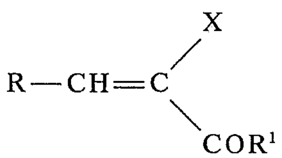

(II)

in welcher R, $R^1$ und X die oben angegebene Bedeutung haben, mit Ammoniak und $\beta$-Ketocarbonsäureestern der Formel V

$$R^2—CO—CH_2—COO—A—CN \qquad (V)$$

in welcher $R^2$ und A die oben angegebene Bedeutung haben, gegebenenfalls in Gegenwart inerter organischer Lösungsmittel umsetzt oder

C) Yliden-$\beta$-dicarbonylverbindungen der Formel VI

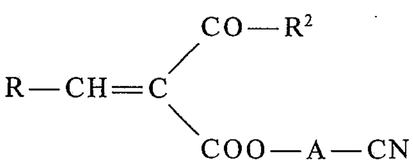

(VI)

in welcher R, $R^2$ und A die oben angegebene Bedeutung haben, mit Enaminoverbindungen der Formel VII

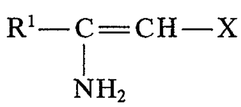

(VII)

in welcher $R^1$ und X die oben angegebene Bedeutung haben, gegebenenfalls in Gegenwart inerter organischer Lösungsmittel zur Reaktion bringt, oder

D) Yliden-$\beta$-dicarbonylverbindungen der Formel VI

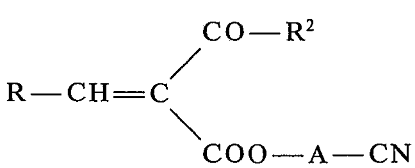

(VI)

in welcher R, $R^2$ und A die oben angegebene Bedeutung haben, mit Ammoniak und Keto-Derivaten der Formel VIII

$$R^1—CO—CH_2—X \qquad (VIII)$$

in welcher $R^1$ und X die oben angegebene Bedeutung haben, gegebenenfalls in Gegenwart inerter organischer Lösungsmittel umsetzt oder

E) Aldehyde der Formel IX

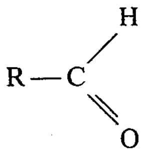

(IX)

in welcher R die oben angegebene Bedeutung hat, mit Enaminoverbindungen der Formel VII

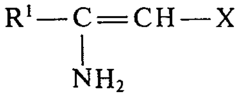

(VII)

in welcher $R^1$ und X die oben angegebene Bedeutung haben, und $\beta$-Ketocarbonsäureestern der Formel V

$$R^2—CO—CH_2—COO—A—CN \qquad (V)$$

in welcher $R^2$ und A die oben angegebene Bedeutung haben, gegebenenfalls in Gegenwart inerter organischer Lösungsmittel umsetzt, oder

F) Aldehyde der Formel IX

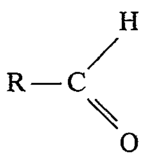

(IX)

in welcher R die oben angegebene Bedeutung hat, mit Enaminocarbonsäureestern der Formel III

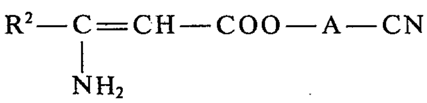

(III)

in welcher $R^2$ und A die oben angegebene Bedeutung haben, und Keto-Derivaten der Formel VIII

$$R^1—CO—CH_2—X \qquad (VIII)$$

in welcher $R^1$ und X die oben angegebene Bedeutung haben, gegebenenfalls in Gegenwart inerter organischer Lösungsmittel

bei Temperaturen zwischen 20 und 150° C umsetzt.

Die erfindungsgemäßen 1,4-Dihydropyridin-Derivate besitzen wertvolle pharmakologische Eigenschaften. Aufgrund ihrer kreislaufbeeinflussenden Wirkung können sie als antihypertensive Mittel, als periphere und cerebrale Vasodilatatoren sowie als Coronartherapeutika Verwendung finden und sind somit als Bereicherung der Pharmazie anzusehen.

Die erfindungsgemäßen Verbindungen wurden mit chemisch ähnlichen Verbindungen aus der deutschen Offenlegungsschrift 2 508 181 verglichen. Die erfindungsgemäßen Verbindungen bewirken bei deutlich niedriger Dosierung einen signifikanten Anstieg der Sauerstoffsättigung (um 20 bis 30%), der deutlich über der Wirkung der bekannten Verbindungen, welche sich nur in der Bedeutung des Restes A—CN unterscheiden, liegt. Diese Verdopplung der Wirkungsstärke ist überraschend und aus dem Stand der Technik nicht herleitbar.

Verfahrensvariante A

Gemäß Verfahren A wird eine Ylidenverbindung der Formel II

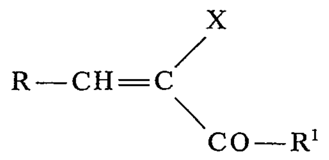

(II)

mit einem Enaminocarbonsäureester der Formel III

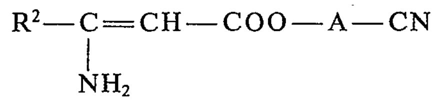

(III)

zur Reaktion gebracht.

Die als Ausgangsstoffe verwendeten Ylidenverbindungen der Formel II sind literaturbekannt oder können nach literaturbekannten Methoden hergestellt werden (vgl. z. B. G. Jones, »The Knoevenagel Condensation« in Org. Reactions, Vol., XV, 204 ff [1967], und G. Beck und D. Günther, Chem. Ber., 106, 2758 [1973]).

Die als Ausgangsstoffe verwendeten Enaminocarbonsäureester der Formel III können nach literaturbekannten Methoden hergestellt werden (vgl. A. C. Cope, J. Amer. Chem. Soc., 67, 1017 [1945]).

Als Verdünnungsmittel kommen alle inerten organischen Lösungsmittel in Frage. Hierzu gehören vorzugsweise Alkohole wie Äthanol, Methanol, Isopropanol, Äther wie Dioxan, Diäthyläther, Tetrahydrofuran, Glykolmonomethyläther, Glykoldimethyläther oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150°C, vorzugsweise zwischen 20 und 100°C, insbesondere bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens wird ein Mol der Ylidenverbindung der Formel II mit einem Mol Enaminocarbonsäureester der Formel III in einem geeigneten Lösungsmittel zur Reaktion gebracht. Die Isolierung und Reinigung der erfindungsgemäßen Substanzen erfolgt vorzugsweise derart, daß man das Lösungsmittel im Vakuum abdestilliert und den gegebenenfalls erst nach Eiskühlung kristallin erhaltenen Rückstand aus einem geeigneten Lösungsmittel umkristallisiert.

Verfahrensvariante B

Gemäß Verfahren B wird eine Ylidenverbindung der Formel II

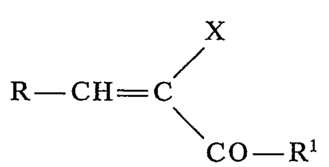

(II)

mit Ammoniak und einem $\beta$-Ketocarbonsäureester der Formel V

$$R^2—CO—CH_2—COO—A—CN \quad (V)$$

zur Reaktion gebracht.

Die als Ausgangsstoffe verwendeten $\beta$-Ketocarbonsäureester der Formel V können nach literaturbekannten Methoden hergestellt werden (z. B. D. Borrmann, »Umsetzung von Diketen mit Alkoholen, Phenolen und Mercaptanen«, in Houben—Weyl, Methoden der Organischen Chemie, Vol. VII/4, 230 ff [1968]).

Als Verdünnungsmittel kommen alle inerten organischen Lösungsmittel in Frage. Hierzu gehören vorzugsweise Alkohole wie Äthanol, Methanol, Isopropanol, Äther wie Dioxan, Diäthyläther, Tetrahydrofuran, Glykolmonomethyläther, Glykoldimethyläther oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen

arbeitet man zwischen 20 und 150° C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens werden die an der Reaktion beteiligten Stoffe der Formeln II, IV und V jeweils in molaren Mengen eingesetzt. Die erfindungsgemäßen Verbindungen können leicht durch Umkristallisation aus einem geeigneten Lösungsmittel gereinigt werden.

### Verfahrensvariante C

Gemäß Verfahren C wird eine Yliden-$\beta$-dicarbonylverbindung der Formel VI

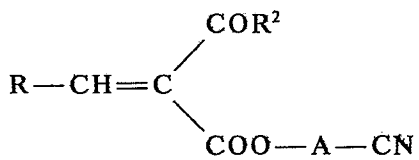

(VI)

mit einer Enaminoverbindung der Formel VII

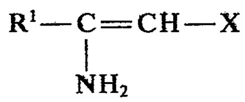

(VII)

zur Reaktion gebracht.

Die als Ausgangsstoffe verwendeten Yliden-$\beta$-dicarbonylverbindungen der Formel VI können nach literaturbekannten Methoden hergestellt werden (vgl. z. B. G. Jones, »The Knoevenagel Condensation« in Org. Reactions, Vol. XV, 204 ff [1967]).

Die als Ausgangsstoffe verwendeten Enaminoverbindungen der Formel VII sind literaturbekannt oder können nach literaturbekannten Methoden hergestellt werden (vgl. A. C. Cope, J. Amer. Chem. Soc., 67, 1017 [1945], N. Guruswamy et al., Indian J. Chem., 11, 882 [1973]).

Als Verdünnungsmittel kommen alle inerten organischen Lösungsmittel in Frage. Hierzu gehören vorzugsweise Alkohole wie Äthanol, Methanol, Isopropanol, Äther wie Dioxan, Diäthyläther, Tetrahydrofuran, Glykolmonomethyläther, Glykoldimethyläther oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150° C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens wird ein Mol der Yliden-$\beta$-dicarbonylverbindung der Formel VI mit einem Mol Enaminoverbindung der Formel VII in einem geeigneten Lösungsmittel zur Reaktion gebracht.

### Verfahrensvariante D

Gemäß Verfahren D wird eine Yliden-$\beta$-dicarbonylverbindung der Formel VI

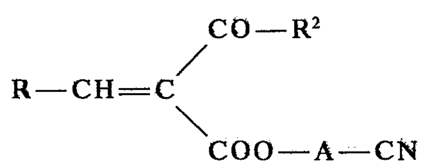

(VI)

mit Ammoniak und einem Keto-Derivat der Formel VIII

$$R^1—CO—CH_2—X \qquad (VIII)$$

zur Reaktion gebracht.

Die als Ausgangssubstanz eingesetzten Keto-Derivate der Formel VIII sind literaturbekannt oder können nach literaturbekannten Methoden hergestellt werden (vgl. z. B. D. Borrmann, »Umsetzung von

Diketen mit Alkoholen, Phenolen und Mercaptanen«, in Houben—Weyl, Methoden der Organischen Chemie, Vol. VII/4, 230 ff [1968]; H. O. House und J. K. Larson, J. Org. Chem., 33, 61 [1968]).

Als Verdünnungsmittel kommen alle inerten organischen Lösungsmittel in Frage. Hierzu gehören vorzugsweise Alkohole wie Äthanol, Methanol, Isopropanol, Äther, wie Dioxan, Diäthyläther, Tetrahydrofuran, Glykolmonomethyläther, Glykoldimethyläther oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150°C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens werden die an der Reaktion beteiligten Stoffe der Formel VI, IV und VIII jeweils in molaren Mengen zugesetzt.

Die erfindungsgemäßen Verbindungen können leicht durch Umkristallisation aus einem geeigneten Lösungsmittel gereinigt werden.

Verfahrensvariante E

Gemäß Verfahren E wird ein Aldehyd der Formel IX

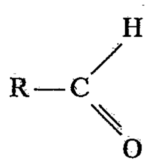

(IX)

mit einer Enaminoverbindung der Formel VII

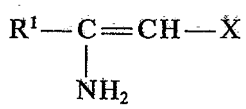

(VII)

und einem $\beta$-Keto-carbonsäureester der Formel V

$$R^2—CO—CH_2—COO—A—CN \qquad (V)$$

zur Reaktion gebracht.

Die als Ausgangsstoffe verwendeten Aldehyde der Formel IX sind literaturbekannt oder können nach literaturbekannten Methoden hergestellt werden (vgl. z. B. E. Mosettig, Org. Reactions VIII, 218 ff [1954]).

Als Verdünnungsmittel kommen alle inerten organischen Lösungsmittel in Frage. Hierzu gehören vorzugsweise Alkohole wie Äthanol, Methanol, Isopropanol, Äther wie Dioxan, Diäthyläther, Tetrahydrofuran, Glykolmonomethyläther, Glykoldimethyläther oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150°C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens werden die an der Reaktion beteiligten Stoffe der Formeln IX, VII und V jeweils in molaren Mengen eingesetzt.

Verfahrensvariante F

Gemäß Verfahren F wird ein Aldehyd der Formel IX

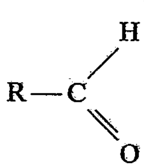

(IX)

mit einem Enaminocarbonsäureester der Formel III

$$R^2—\underset{NH_2}{\underset{|}{C}}=CH—COO—A—CN \qquad (III)$$

und einem Keto-Derivat der Formel VIII

$$R^1—CO—CH_2—X \qquad (VIII)$$

zur Reaktion gebracht.

Als Verdünnungsmittel kommen alle inerten organischen Lösungsmittel in Frage. Hierzu gehören vorzugsweise Alkohole wie Äthanol, Methanol, Isopropanol, Äther wie Dioxan, Diäthyläther, Tetrahydrofuran, Glykolmonomethyläther, Glykoldimethyläther, oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150°C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Bei der Durchführung des erfindungsgemäßen Verfahrens werden die an der Reaktion beteiligten Stoffe der Formeln IX, III und VIII jeweils etwa in molaren Mengen eingesetzt.

Die vorstehenden Herstellungsverfahren sind lediglich zur Verdeutlichung angegeben, und die Herstellung der Verbindungen der Formel I ist nicht auf diese Verfahren beschränkt, sondern jede Modifikation dieser Verfahren ist in gleicher Weise für die Herstellung der erfindungsgemäßen Verbindungen anwendbar.

Je nach Wahl der Ausgangssubstanzen können die erfindungsgemäßen Verbindungen in stereoisomeren Formen existieren, die sich entweder wie Bild und Spiegelbild (Enantiomere) oder die sich nicht wie Bild und Spiegelbild (Diastereomere) verhalten. Sowohl die Antipoden als auch die Racemformen als auch die Diastereomerengemische sind Gegenstand der vorliegenden Erfindung. Die Racemformen lassen sich ebenso wie die Diastereomeren in bekannter Weise in die stereoisomer einheitlichen Bestandteile trennen (vgl. z. B. E. L. Eliel, Stereochemistry of Carbon Compounds, McGraw—Hill, 1962).

Die neuen Verbindungen haben ein breites und vielseitiges pharmakologisches Wirkungsspektrum. Im einzelnen konnten im Tierexperiment folgende Hauptwirkungen nachgewiesen werden:

1. Die Verbindungen bewirken bei parenteraler, oraler und perlingualer Zugabe eine deutliche und langanhaltende Erweiterung der Coronargefäße. Diese Wirkung auf die Coronargefäße wird durch einen gleichzeitigen Nitrit-ähnlichen herzentlastenden Effekt verstärkt.
   Sie beeinflussen bzw. verändern den Herzstoffwechsel im Sinne einer Energieersparnis.
2. Die Erregbarkeit des Reizbildungs- und Erregungsleitungssystems innerhalb des Herzens wird herabgesetzt, so daß eine in therapeutischen Dosen nachweisbare Antiflimmerwirkung resultiert.
3. Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten (wie z. B. dem Zentralnervensystem) manifestieren.
4. Die Verbindungen senken den Blutdruck von normotonen und hypertonen Tieren und können somit als anithypertensive Mittel verwendet werden.
5. Die Verbindungen haben stark muskulär-spasmolytische Wirkungen, diese an der glatten Muskulatur des Magens, Darmtraktes, des Urogenitaltraktes und des Respirationssystems deutlich werden.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nichttoxischer pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d. h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z. B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nichttoxische organische Lösungsmittel, wie Paraffine (z. B. Erdölfraktionen), pflanzliche Öle (z. B. Erdnuß-/Sesamöl), Alkohole (z. B. Äthylalkohol, Glycerin), Glykole (z. B. Propylenglykol,

Polyäthylenglykol), feste Trägerstoffe, wie z. B. natürliche Gesteinsmehle (z. B. Kaoline, Tonerden, Talkum, Kreide), synthetische Gesteinsmehle (z. B. hochdisperse Kieselsäure, Silikate), Zucker (z. B. Roh-, Milch- und Traubenzucker), Emulgiermittel (z. B. Polyoxyäthylen-Fettsäure-Ester, Polyoxyäthylen-Fettalkohol-Äther, Alkylsulfonate und Arylsulfonate), Dispergiermittel (z. B. Lignin, Sulfitablaufen, Methylcellulose, Stärke und Polyvinylpyrrolidon) und Gleitmittel (z. B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,05 bis 5 mg/kg Körpergewicht pro Tag zur Erzielung wirksamer Ergebnisse zu verabreichen, und bei oraler Applikation beträgt die Dosierung etwa 0,05 bis 20 mg/kg, vorzugsweise 0,5 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. deren Art von dessen Formulierung und dem Zeitpunkt bzw. Intervall, zu welchem die Verabreichtung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

## Herstellungsbeispiele

### Beispiel 1

1,4-Dihydro-2,6-dimethyl-4-(2-trifluormethylphenyl)-pyridin-3,5-dicarbonsäure-äthyl-(2-cyanoäthyl)-ester

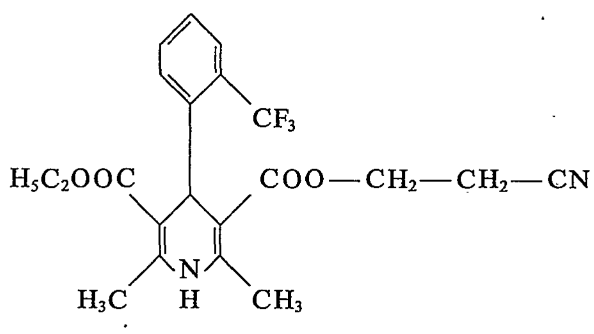

### Verfahrensvariante A

Eine Lösung von 18,6 g (75 mMol) 2′-Trifluormethylbenzylidenacetessigsäureäthylester und 11,6 g (75 mMol) β-Aminocrotonsäure-(2-cyanoäthyl)-ester in 120 ml Äthanol wurde 12 Stunden zum Sieden erhitzt. Nach Erkalten der Reaktionsmischung wurde das Lösungsmittel im Vakuum abdestilliert, der ölige Rückstand mit wenig Äther versetzt und zur Kristallisation gebracht. Das Produkt wurde abgesaugt und aus Äthanol umkristallisiert.

Schmelzpunkt: 126° C
Ausbeute: 21 g (66%)

Verfahrensvariante E

Eine Lösung von 13 g (75 mMol) 2-Trifluormethylbenzaldehyd, 9,7 g (75 mMol) $\beta$-Aminocrotonsäureäthylester und 11,6 g (75 mMol) Acetessigsäure-(2-cyanoäthyl)-ester in 120 ml Äthanol wurde 15 Stunden unter Rückfluß erhitzt. Anschließend wurde das Lösungsmittel im Vakuum abdestilliert, der ölige Rückstand mit wenige Äther versetzt und zur Kristallisation gebracht. Das Produkt wurde abgesaugt und aus Äthanol umkristallisiert.

Schmelzpunkt: 126° C
Ausbeute: 17,5 g (55%)

Analog Beispiel 1 wurden folgende Verbindungen erhalten:

Tabelle 1:

| Nr. | Formel | Lösungsmittel | Schmelzpunkt | Ausbeute |
|---|---|---|---|---|
| 2 | 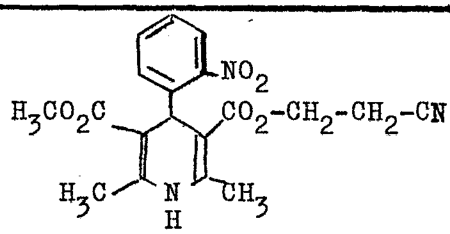 | Methanol | 154°C | 48 % |
| 3 | 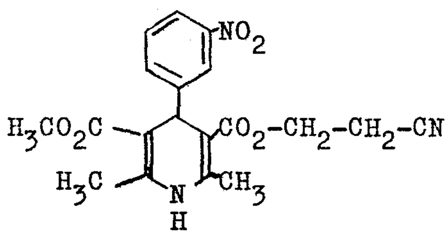 | Methanol | 135°C | 55 % |
| 4 | 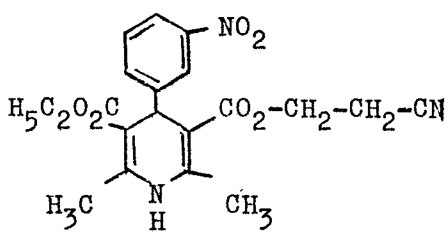 | Äthanol | 152°C | 63 % |
| 5 | 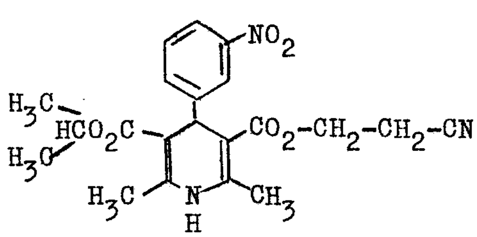 | Äthanol | 141°C | 68 % |
| 6 | 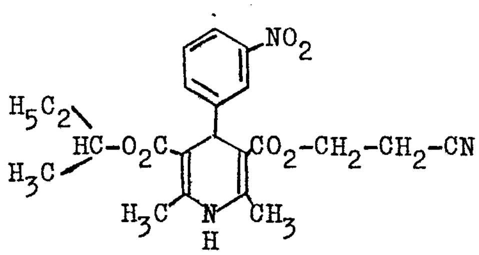 | Dimethyl-formamid | 106°C | 60 % |

Tabelle 1: (Fortsetzung)

| Nr. | Formel | Lösungsmittel | Schmelzpunkt | Ausbeute |
|---|---|---|---|---|
| 7 | 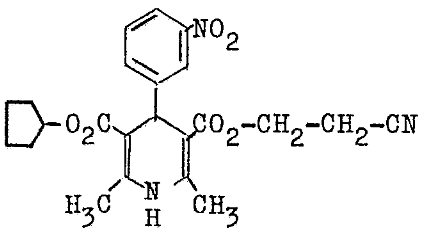 | Äthanol | 131°C | 66 % |
| 8 | 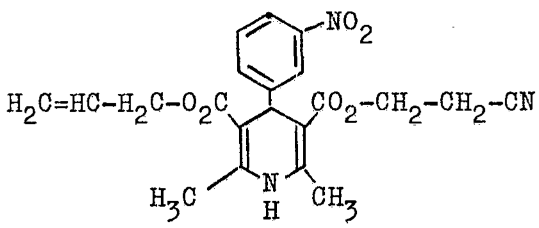 | Äthanol | 142°C | 45 % |
| 9 | 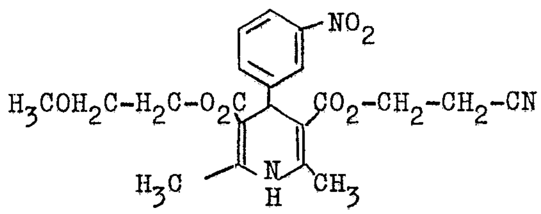 | Äthanol | 141°C | 58 % |
| 10 | 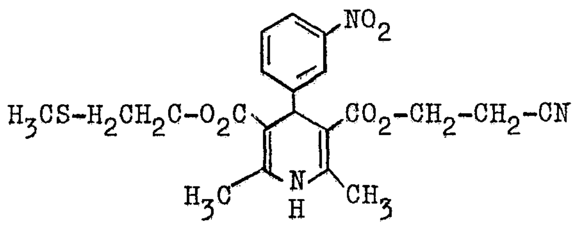 | Äthanol | 152°C | 71 % |
| 11 | 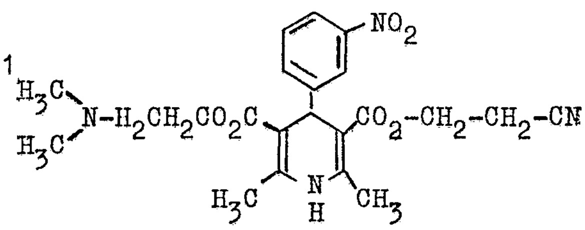 | Äthanol | Öl | 45 % |
| 12 | 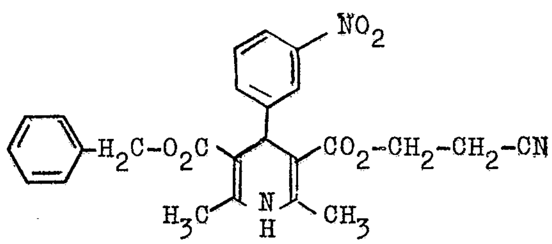 | HMPT | 117°C | 68 % |
| 13 | 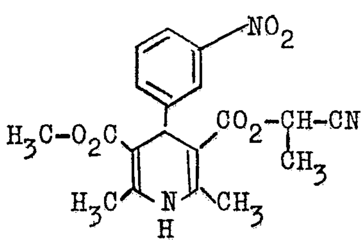 | Iso-propanol | 183°C | 45 % |

Tabelle 1: (Fortsetzung)

| Nr | Formel | Lösungsmittel | Schmelzpunkt | Ausbeute |
|---|---|---|---|---|
| 14 | 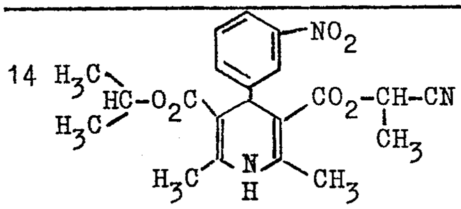 | Äthanol | 123°C | 40 % |
| 15 | 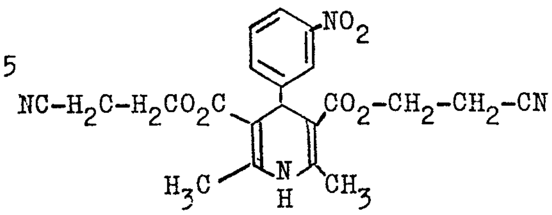 | Äthanol | 166°C | 65 % |
| 16 | 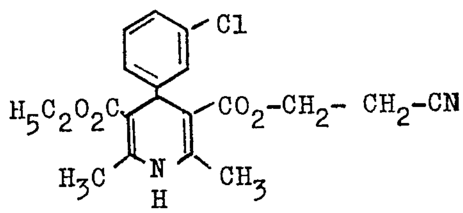 | Äthanol | Öl | 51 % |
| 17 | 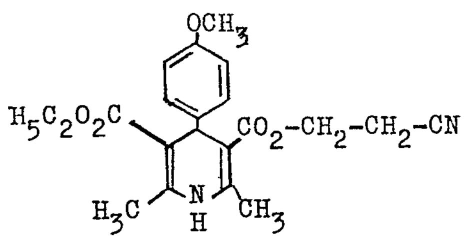 | Äthanol | 132°C | 51 % |
| 18 | 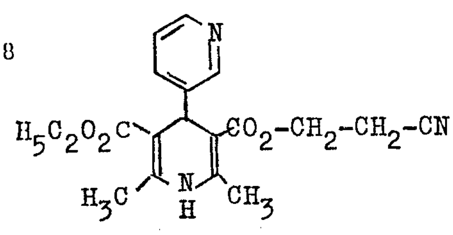 | Äthanol | 195°C | 56 % |
| 19 | 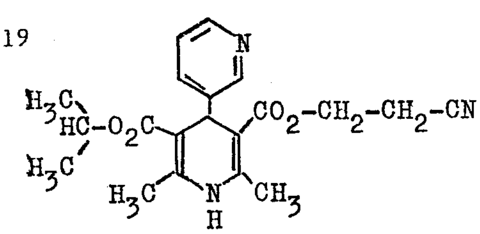 | Isopropanol | 192°C | 49 % |
| 20 | 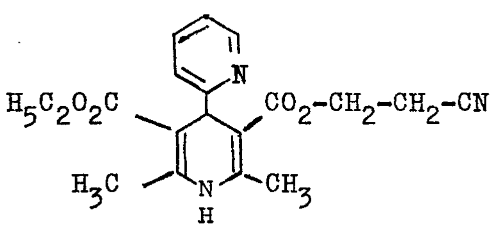 | Äthanol | 169°C | 70 % |

**Patentansprüche**

1. 1,4-Dihydropyridine mit funktionalisierten Estergruppen der allgemeinen Formel I

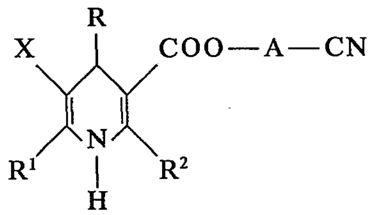

(I)

in welcher

R für einen Phenylrest steht, der durch Trifluormethyl, Nitro, Halogen oder Cyano substituiert ist oder für einen Pyridylrest steht,

$R^1$ und $R^2$ gleich oder verschieden sind und jeweils für Methyl oder Ethyl stehen,

A für eine geradkettige oder verzweigte Alkylengruppe mit bis zu 4 Kohlenstoffatomen steht,

X für die Gruppe $-COOR^7$ steht, wobei $R^7$ einen geradkettigen, verzweigten, cyclischen, gesättigten oder ungesättigten Kohlenwasserstoffrest mit bis zu 6 Kohlenstoffatomen bedeutet, der gegebenenfalls durch 1 Sauerstoff- oder Schwefelatom in der Kette unterbrochen ist und der gegebenenfalls substituiert ist durch Trifluormethyl, Phenyl oder Di-($C_1-C_4$)alkylamino,
oder
für die Gruppe $-COO-A'-CN$ steht, wobei diese Gruppe mit $-COO-A-CN$ gleich oder verschieden ist und A' für eine geradkettige oder verzweigte Alkylengruppe mit bis zu 4 Kohlenstoffatomen steht.

2. Verfahren zur Herstellung von Verbindungen der allgemeinen Formel I

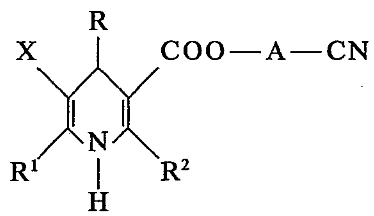

(I)

in welcher

R für einen Phenylrest steht, der durch Trifluormethyl, Nitro, Halogen oder Cyano substituiert ist oder für einen Pyridylrest steht,

$R^1$ und $R^2$ gleich oder verschieden sind und jeweils für Methyl oder Ethyl stehen,

A für eine geradkettige oder verzweigte Alkylengruppe mit bis zu 4 Kohlenstoffatomen steht,

X für die Gruppe $-COOR^7$ steht, wobei $R^7$ einen geradkettigen, verzweigten, cyclischen, gesättigten oder ungesättigten Kohlenwasserstoffrest mit bis zu 6 Kohlenstoffatomen bedeutet, der gegebenenfalls durch 1 Sauerstoff- oder Schwefelatom in der Kette unterbrochen ist und der gegebenenfalls substituiert ist durch Trifluormethyl, Phenyl oder Di-($C_1-C_4$)alkylamino,
oder
für die Gruppe $-COO-A'-CN$ steht, wobei diese Gruppe mit $-COO-A-CN$ gleich oder verschieden ist und A' für eine geradkettige oder verzweigte Alkylengruppe mit bis zu 4 Kohlenstoffatomen steht,

dadurch gekennzeichnet, daß man

A) Ylidenverbindungen der Formel II

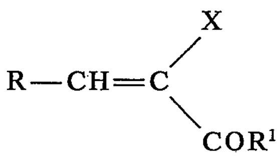

(II)

in welcher R, $R^1$ und X die oben angegebene Bedeutung haben, mit Enaminocarbonsäureestern der Formel III

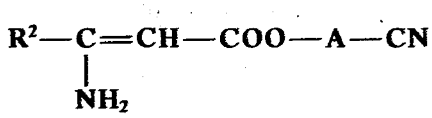

(III)

in welcher $R^2$ und A die oben angegebene Bedeutung haben, gegebenenfalls in Gegenwart inerter organischer Lösungsmittel umsetzt oder

B) Ylidenverbindungen der Formel II

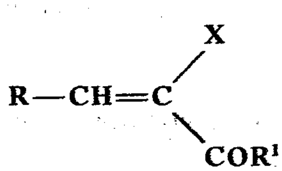

(II)

in welcher R, $R^1$ und X die oben angegebene Bedeutung haben, mit Ammoniak und β-Ketocarbonsäureestern der Formel V

$$R^2—CO—CH_2—COO—A—CN \quad (V)$$

in welchen $R^2$ und A die oben angegebene Bedeutung haben, gegebenenfalls in Gegenwart inerter organischer Lösungsmittel umsetzt oder

C) Yliden-β-dicarbonylverbindungen der Formel VI

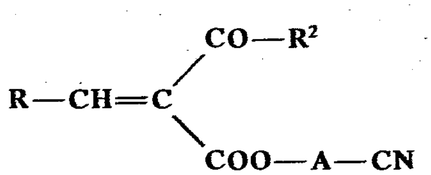

(VI)

in welcher R, $R^2$ und A die oben angegebene Bedeutung haben, mit Enaminoverbindungen der Formel VII

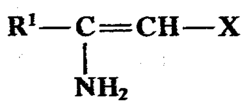

(VII)

in welcher $R^1$ und X die oben angegebene Bedeutung haben, gegebenenfalls in Gegenwart inerter organischer Lösungsmittel zur Reaktion bringt, oder

D) Yliden-β-dicarbonylverbindungen der Formel VI

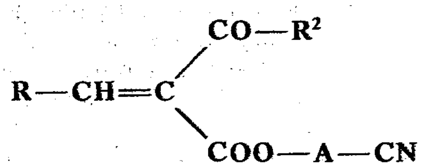

(VI)

in welcher R, $R^2$ und A die oben angegebene Bedeutung haben, mit Ammoniak und Keto-Derivaten der Formel VIII

$$R^1—CO—CH_2—X \quad (VIII)$$

in welcher $R^1$ und X die oben angegebene Bedeutung haben, gegebenenfalls in Gegenwart inerter organischer Lösungsmittel umsetzt oder

E) Aldehyde der Formel IX

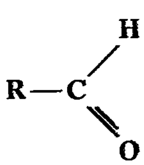

(IX)

in welcher R die oben angegebene Bedeutung hat, mit Enaminoverbindungen der Formel VII

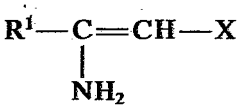

(VII)

in welcher $R^1$ und X die oben angegebene Bedeutung haben, und $\beta$-Ketocarbonsäureestern der Formel V

$$R^2—CO—CH_2—COO—A—CN \qquad (V)$$

in welcher $R^2$ und A die oben angegebene Bedeutung haben, gegebenenfalls in Gegenwart inerter organischer Lösungsmittel umsetzt, oder

F) Aldehyde der Formel IX

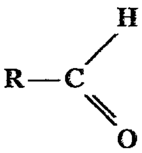

(IX)

in welcher R die oben angegebene Bedeutung hat, mit Enaminocarbonsäureestern der Formel III

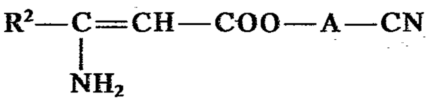

(III)

in welcher $R^2$ und A die oben angegebene Bedeutung haben, und Keto-Derivaten der Formel VIII

$$R^1—CO—CH_2—X \qquad (VIII)$$

in welcher $R^1$ und X die oben angegebene Bedeutung haben, gegebenenfalls in Gegenwart inerter organischer Lösungsmittel bei Temperaturen zwischen 20 und 150°C umsetzt.

**3. Arzneimittel, enthaltend mindestens eine Verbindung der allgemeinen Formel I gemäß Anspruch 1.**

**4. Verfahren zur Herstellung von Arzneimitteln, dadurch gekennzeichnet, daß man mindestens eine Verbindung der allgemeinen Formel I gemäß Anspruch 1 gegebenenfalls unter Verwendung von Hilfs- und Trägerstoffen in eine geeignete Applikationsform überführt.**

## Claims

1. 1,4-Dihydropyridines with functionalised ester groups of the general formula I

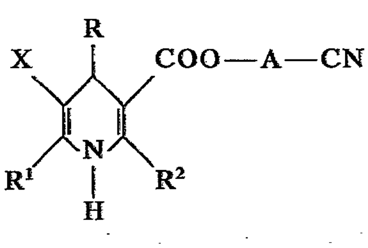

(I)

in which

R represents a phenyl radical which is substituted by trifluoromethyl, nitro, halogen or cyano, or represents a pyridyl radical,

$R^1$ and $R^2$ are identical or different and each represents methyl or ethyl,

A represents a straight-chain or branched alkylene group with up to 4 carbon atoms,

X represents the group $-COOR^7$, wherein $R^7$ denotes a straight-chain, branched or cyclic, saturated or unsaturated hydrocarbon radical with up to 6 carbon atoms, which is optionally interrupted in

the chain by 1 oxygen or sulphur atom and which is optionally substituted by trifluoromethyl, phenyl or di-($C_1-C_4$)-alkylamino, or represents the group $-COO-A'-CN$, this group being identical or different to $COO-A-CN$, and A' representing a straight-chain or branched alkylene group with up to 4 carbon atoms.

2. Process for the production of compounds of the general formula I

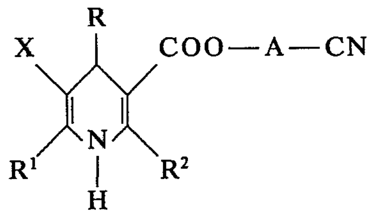

(I)

in which

R represents a phenyl radical which is substituted by trifluoromethyl, nitro, halogen or cyano, or represents a pyridyl radical,

$R^1$ and $R^2$ are identical or different and each represents methyl or ethyl,

A represents a straight-chain or branched alkylene group with up to 4 carbon atoms,

X represents the group $-COOR^7$, wherein $R^7$ denotes a straight-chain, branched or cyclic, saturated or unsaturated hydrocarbon radical with up to 6 carbon atoms, which is optionally interrupted in the chain by 1 oxygen or sulphur atom and which is optionally substituted by trifluoromethyl, phenyl or di-($C_1-C_4$)-alkylamino, or represents the group $-COO-A'-CN$, this group being identical or different to $COO-A-CN$, and A' representing a straight-chain or branched alkylene group with up to 4 carbon atoms,

characterised in that

A) ylidene compounds of the formula II

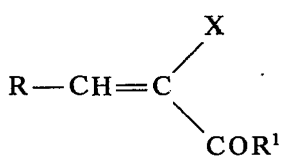

(II)

in which R, $R^1$ and X have the meaningsindicated above, are reacted with enaminocarboxylic acid esters of the formula III

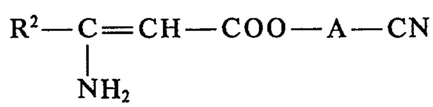

(III)

in which $R^2$ and A have the meaningsindicated above, if appropriate in the presence of inert organic solvents or

B) ylidene compounds of the formula II

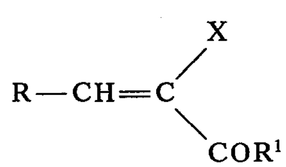

(II)

in which R, $R^1$ and X have meaningsindicated above, are reacted with ammonia and $\beta$-ketocarboxylic acid esters of the formula V

$$R^2-CO-CH_2-COO-A-CN \qquad (V)$$

in which $R^2$ and A have the meaningsindicated above, if appropriate in the presence of inert organic silvents, or

C) ylidene-$\beta$-dicarbonyl compounds of the formula VI

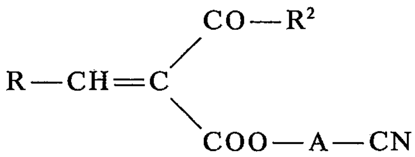 (VI)

in which R, $R^2$ and A have the meaningsindicated above, are reacted with enamino compounds of the formula VII

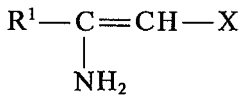 (VII)

in which $R^1$ and X have the meaningsindicated above, if appropriate in the presence of inert organic solvents, or

D) ylidene-$\beta$-dicarbonyl compounds of the formula VI

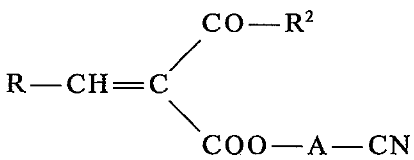 (VI)

in which R, $R^2$ and A have the meaningsindicated above, are reacted with ammonia and keto derivatives of the formula VIII

$$R^1—CO—CH_2—X \quad (VIII)$$

in which $R^1$ and X have the meaningsindicated above, if appropriate in the presence of inert organic solvents, or

E) aldehydes of the formula IX

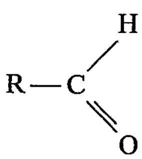 (IX)

in which R has the meaning indicated above, are reacted with enamino compounds of the formula VII

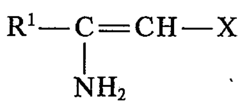 (VII)

in which $R^1$ and X have the meaningsindicated above, and $\beta$-ketocarboxylic acid esters of the formula V

$$R^2—CO—CH_2—COO—A—CN \quad (V)$$

in which $R^2$ and A have the meaningsindicated above, if appropriate in the presence of inert organic solvents, or

F) aldehydes of the formula IX

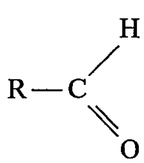 (IX)

in which R has the meaning indicated above, are reacted with enaminocarboxylic acid esters of the formula III

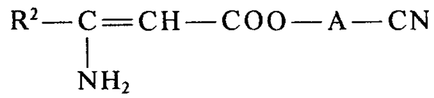 (III)

in which $R^2$ and A have the meaningsindicated above, and keto derivatives of the formula VIII

$$R^1—CO—CH_2—X \qquad (VIII)$$

in which $R^1$ and X have the meaningsindicated above, if appropriate in the presence of inert organic solvents, at temperatures between 20 and 150°C.

3. Medicaments containing at least one compound of the general formula I according to Claim 1.

4. Process for producing medicaments, characterised in that at least one compound of the general formula I according to Claim 1 is converted into a suitable application form, if appropriate using auxiliaries and excipients.

## Revendications

1. 1,4-dihydropyridines comportant des groupes ester fonctionnalisés de formule générale I:

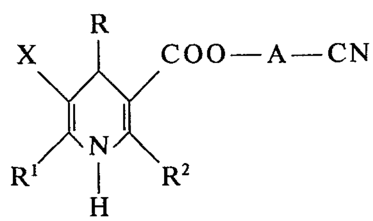 (I)

dans laquelle

R représente un groupe phényle substitué par un groupe trifluorométhyle, un groupe nitro, un atome d'halogène ou par un groupe cyano, ou encore il représente un groupe pyridyle;

$R^1$ et $R^2$ sont identiques ou différents et représentent chacun un groupe méthyle ou un groupe éthyle;

A représente un groupe alkylène à chaîne droite ou ramifiée contenant jusqu'à 4 atomes de carbone;

X représente le groupe $-COOR^7$, $R^7$ représentant un radical d'hydrocarbure à chaîne droite, à chaîne ramifiée, cyclique, saturé ou insaturé et contenant jusqu'à 6 atomes de carbone, la chaîne de ce radical étant éventuellement interrompue par un atome d'oxygène ou de soufre, tandis que ce radical est éventuellement substitué par un groupe trifluorométhyle, phényle ou di-alkyl(en $C_1-C_4$)amino, ou encore il représente le groupe $-COO-A'-CN$, ce groupe étant identique à ou différent du groupe $-COO-A-CN$, tandis que A' représente un groupe alkylène à chaîne droite ou ramifiée contenant jusqu'à 4 atomes de carbone.

2. Procédé de préparation de composés de formule générale I:

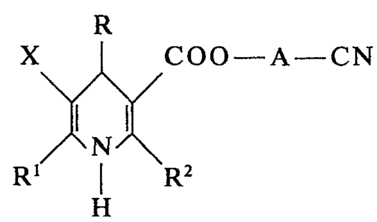 (I)

dans laquelle

R représente un groupe phényle substitué par un groupe trifluorométhyle, un groupe nitro, un atome d'halogène ou par un groupe cyano, ou encore il représente un groupe pyridyle;

$R^1$ et $R^2$ sont identiques ou différents et représentent chacun un groupe méthyle ou un groupe éthyle;

A représente un groupe alkylène à chaîne droite ou ramifiée contenant jusqu'à 4 atomes de carbone;

X représente le groupe $-COOR^7$, $R^7$ représentant un radical d'hydrocarbure à chaîne droite, à chaîne ramifiée, cyclique, saturé ou insaturé et contenant jusqu'à 6 atomes de carbone, la chaîne de ce radical étant éventuellement interrompue par un atome d'oxygène ou de soufre, tandis que ce radical est éventuellement substitué par un groupe trifluorométhyle, phényle ou di-alkyl(en

$C_1-C_4$)amino, ou encore il représente le groupe $-COO-A'-CN$, ce groupe étant identique à ou différent du groupe $-COO-A-CN$, tandis que A' représente un groupe alkylène à chaîne droite ou ramifiée contenant jusqu'à 4 atomes de carbone,

caractérisé en ce que:

A) on fait réagir des composés ‹ylidène› de formule II:

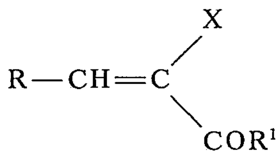

(II)

dans laquelle R, $R^1$ et X ont les significations indiquées ci-dessus, avec des esters d'acides énamino-carboxyliques de formule III:

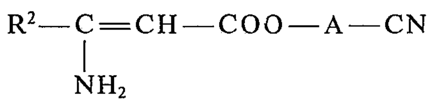

(III)

dans laquelle $R^2$ et A ont les significations indiquées ci-dessus, eventuellement en présence de solvants organiques inertes, ou

B) on fait réagir des composés ‹ylidène› de formule II:

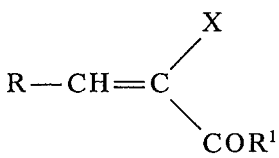

(II)

dans laquelle R, $R^1$ et X ont les significations indiquées ci-dessus, avec de l'ammoniac et des esters d'acides $\beta$-céto-carboxyliques de formule V:

$$R^2-CO-CH_2-COO-A-CN \quad (V)$$

dans laquelle $R^2$ et A ont les significations indiquées ci-dessus, éventuellement en présence de solvants organiques inertes, ou

C) on fait réagir des composés ‹ylidène-$\beta$-dicarbonyle› de formule VI:

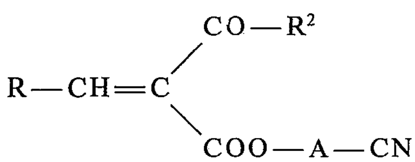

(VI)

dans laquelle R, $R^2$ et A ont les significations indiquées ci-dessus, avec des énamino-composés de formule VII:

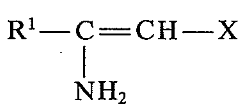

(VII)

dans laquelle $R^1$ et X ont les significations indiquées ci-dessus, éventuellement, en présence de solvant organiques inertes, ou

D) on fait réagir des composés ‹ylidène-$\beta$-dicarbonyle› de formule VI:

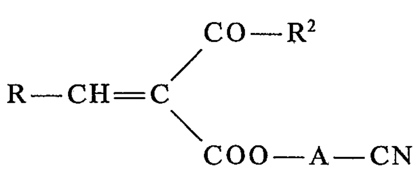

(VI)

dans laquelle R, $R^2$ et A ont les significations indiquées ci-dessus, avec de l'ammonic et des céto-dérivés de formule VIII:

$R^1—CO—CH_2—X$ (VIII)

dans laquelle $R^1$ et X ont les significations indiquées ci-dessus, éventuellement en présence de solvants organiques inertes, ou

E) on fait réagir des aldéhydes de formule IX:

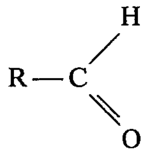

(IX)

dans laquelle R a la significations indiquées ci-dessus, avec des énamino-composés de formule VII:

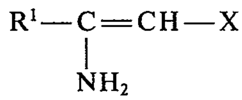

(VII)

dans laquelle $R^1$ et X ont les significations indiquées ci-dessus, ainsi qu'avec des esters d'acides $\beta$-céto-carboxyliques de formule V:

$R^2—CO—CH_2—COO—A—CN$ (V)

dans laquelle $R^2$ et A ont les significations indiquées ci-dessus, éventuellement en présence de solvants organiques inertes, ou

F) on fait réagir des aldéhydes de formule IX:

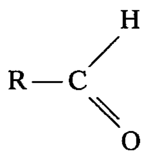

(IX)

dans laquelle R a la significations indiquées ci-dessus, avec des esters d'acides énamino-carboxyliques de formule III:

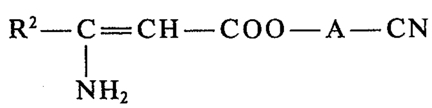

(III)

dans laquelle $R^2$ et A ont les significations indiquées ci-dessus, ainsi qu'avec des céto-dérivés de formule VIII:

$R^1—CO—CH_2—X$ (VIII)

dans laquelle $R^1$ et X ont les significations indiquées ci-dessus, éventuellement en présence de solvants organiques inertes, à des températures comprises entre 20 et 150°C.

**3. Médicament contenant au moins un composé répondant à la formule générale I suivant la revendication 1.**

**4. Procédé de préparation de médicaments, caractérisé en ce qu'on transforme, en une forme d'application appropriée, au moins un composé répondant à la formule générale I suivant la revendication 1 en utilisant éventuellement des substances auxiliaires et des substances supports.**